# FASCICULE DE BREVET EUROPEEN

(11) **EP 3 072 872 B1**
(45) Date de publication et mention de la délivrance du brevet: **26.06.2019**
(21) Numéro de dépôt: 16166230.9
(22) Date de dépôt: 05.05.2010
(51) Int. Cl.: C07C 17/354, C07C 19/08

(54) **PROCEDE DE FABRICATION DU HEXAFLUOROPROPANE**
HERSTELLUNGSVERFAHREN VON HEXAFLUORPROPAN
METHOD FOR MANUFACTURING HEXAFLUOROPROPANE

(30) Priorité: 12.06.2009 FR 0953941
(43) Date de publication de la demande: 28.09.2016
(62) Demande divisionnaire de: 10727102.5
(73) Titulaire: ARKEMA FRANCE, 92700 Colombes (FR)
(72) Inventeur: DEVIC, Michel, 69110 Sainte Foy les Lyons (FR); DOUCET, Nicolas, 57420 Cuvry (FR); WENDLINGER, Laurent, 69510 Soucieu en Jarrest (FR); CAVALLINI, Géraldine, 201702 Shanghai (CN)
(74) Mandataire: Leca, François Michel

(56) Documents cités:
- EP-A1- 1 916 232
- DATABASE WPI Week 200872 Thomson Scientific, London, GB; AN 2008-M14458 XP002564309, & CN 101 148 395 A (ZHEJIANG LANTIAN ENVIRONMENTAL PROTECTIO) 26 mars 2008 (2008-03-26)
- DATABASE CA [Online] CHEMICAL ABSTRACTS SERVICE, COLUMBUS, OHIO, US; 1961, KNUNYANTS, I. L. ET AL: "Reactions of fluoro olefins. XIII. Catalytic hydrogenation of perfluoro olefins", XP002564154, extrait de STN Database accession no. 1961:2127 & KNUNYANTS, I. L. ET AL: "Reactions of fluoro olefins. XIII. Catalytic hydrogenation of perfluoro olefins", IZVESTIYA AKADEMII NAUK SSSR, SERIYA KHIMICHESKAYA 1412-18 CODEN: IASKA6; ISSN: 0002-3353, 1960,

## Description

La présente invention concerne un procédé de fabrication du 1,1,1,2,3,3-hexafluoropropane par hydrogénation de l'hexafluoropropène.

Le document de Knunyants et al., Journal of the USSR Academy of Sciences, Chemistry Department, «reactions of fluoro-olefins», report 13, "catalytic hydrogenation of perfluoro-olefins", 1960, décrit l'hydrogénation sensiblement quantitative de l'hexafluoropropène (HFP) sur un catalyseur à base de palladium supporté sur l'alumine, la température passant de 20°C à 50°C, puis étant maintenue à cette valeur.

Le 1,1,1,2,3,3-hexafluoropropane est utile pour la fabrication du 1,2,3,3,3-pentafluoropropène.

Les essais de l'art antérieur précité ont été effectués à l'échelle laboratoire et les documents sont totalement muets sur la durée de vie de ces catalyseurs.

La réaction d'hydrogénation telles que décrite précédemment est fortement exothermique et pose des problèmes à l'échelle industrielle. Cette forte exothermicité est néfaste à la durée de vie du catalyseur.

La présence d'un composé, autre que les réactifs, dans le flux réactionnel peut également être à l'origine d'une désactivation rapide du catalyseur.

Par ailleurs, le document EP 1916232 propose une réaction d'hydrogénation multiétagée d'un composé oléfinique pour obtenir une conversion et une sélectivité élevée. L'exemple 1 décrit la réaction d'hydrogénation étagée de l'hexafluoropropène en présence d'un catalyseur supporté sur charbon dans quatre réacteurs avec une température du flux gazeux à la sortie du premier réacteur de 66°C, une température de 104°C à la sortie du deuxième réacteur (pour une conversion de 40%), une température de 173°C à la sortie du troisième, une température de 100°C à la sortie du quatrième. Il est prévu des étapes de refroidissement entre les réacteurs avec une température du premier bain de 55°C et celle du deuxième bain de 111°C.

Le procédé tel que décrit dans le document EP 1916232 est coûteux en investissement et en outre, sa mise en oeuvre n'est pas aisée.

Le procédé, selon la présente invention, permet de maîtriser l'exothermicité de la réaction d'hydrogénation tout en gardant une très bonne conversion et sélectivité et/ou réduire la désactivation du catalyseur.

Le procédé selon la présente invention est caractérisé en ce que (i) l'on fait réagir en phase gazeuse de l'hexafluoropropène avec de l'hydrogène en quantité surstoechiométrique en présence d'un catalyseur d'hydrogénation; (ii) l'on recycle au moins 90% en volume de la totalité de l'effluent gazeux issu du réacteur comprenant du 1,1,1,2,3,3-hexafluoropropane, de l'hydrogène non réagi et éventuellement de l'hexafluoropropène non réagi, du 1,1,1,2,3-pentafluoropropane et du 1,1,1,2-tetrafluoropropane et (iii) l'on récupère du 1,1,1,2,3,3-hexafluoropropane de l'autre partie de l'effluent gazeux issu du réacteur éventuellement après une étape de purification, caractérisé en ce que la température à l'entrée du réacteur est comprise entre 30 et 100°C.

De préférence, la température à l'entrée du réacteur est comprise entre 40 et 80° C.

De préférence, l'étape i) est effectuée à une température comprise entre 50 et 200°C.

Le flux gazeux comprenant la boucle de recyclage et les réactifs peuvent être préchauffés avant introduction dans le réacteur.

Le procédé selon la présente invention est de préférence mis en oeuvre avec un rapport molaire hydrogène / HFP compris entre 1 et 50, avantageusement compris entre 2 et 15.

Le temps de contact, défini comme le rapport du volume du lit catalytique sur le débit volumique du flux total dans les conditions normales de température et de pression, est de préférence comprise entre 0,1s et 20s et avantageusement comprise entre 0,5 et 5s.

La réaction d'hydrogénation, selon la présente invention, est de préférence mise en oeuvre à une pression absolue comprise entre 0,5 et 20 bar et avantageusement comprise entre 1 et 5 bar.

L'effluent gazeux à la sortie du réacteur comprend de préférence environ 2 à 99 % en volume du 1,1,1,2,3,3-hexafluoropropane, 0,2 à 98% en volume d'hydrogène, 0 à 10 % du 1,1,1,2,3,3-hexafluoropropène, de 0 à 5% de 1,1,1,2,3 pentafluoropropane et de 0 à 1% de 1,1,1,2-tetrafluoropropane.

Avantageusement, l'effluent gazeux à la sortie du réacteur comprend de 50 à 98 % en volume du 1,1,1,2,3,3-hexafluoropropane, 2 à 50% en volume d'hydrogène, 0 à 0,1% du 1,1,1,2,3,3-hexafluoropropène, de 0 à 1% de 1,1,1,2,3 pentafluoropropane et de 0 à 0,5% de 1,1,1,2-tetrafluoropropane.

Selon le procédé de l'invention on utilise, de préférence un réacteur adiabatique.

La partie de l'effluent gazeux recyclée au réacteur représente, avantageusement au moins 93% en volume. De façon particulièrement préférée, la partie de l'effluent recyclée au réacteur représente entre 94 et 98% en volume de l'effluent total à la sortie du réacteur.

Comme catalyseur, on peut notamment citer ceux à base d'un métal du groupe VIII ou rhénium. Le catalyseur peut être supporté, par exemple sur carbone, alumine, fluorure d'aluminium, etc. ou peut ne pas être supporté, comme le nickel de Raney. Comme métal, on peut utiliser du platine ou du palladium, en particulier du palladium, avantageusement supporté sur carbone ou alumine. On peut aussi associer ce métal avec un autre métal comme l'argent, le cuivre, l'or, le tellure, le zinc, le chrome, le molybdène et le thallium.
De préférence, le catalyseur comprend du palladium éventuellement supporté.

Le catalyseur tout particulièrement préféré selon la présente invention est un catalyseur contenant du palladium supporté sur de l'alumine. La quantité de palladium dans le catalyseur est de préférence comprise entre 0,05 et 10% en poids et avantageusement entre 0,1 et 5%
La surface spécifique du catalyseur est de préférence supérieure à 4 m²/g et d l'alumine utilisé en tant que support catalytique se présente avantageusement sous la forme polymorphique α.

Selon un mode de réalisation 1, l'invention fournit un procédé de fabrication du 1,1,1,2,3,3-hexafluoropropane caractérisé en ce que (i) l'on fait réagir en phase gazeuse de l'hexafluoropropène avec de l'hydrogène en quantité surstoechiométrique à une température comprise entre 50 et 200 °C, de préférence comprise entre 80 et 120 °C, en présence d'un catalyseur d'hydrogénation; (ii) l'on recycle une partie de l'effluent gazeux issu du réacteur comprenant du 1,1,1,2,3,3-hexafluoropropane, de l'hydrogène non réagi et éventuellement de l'hexafluoropropène non réagi, du 1,1,1,2,3-pentafluoropropane et du 1,1,1,2-tetrafluoropropane et (iii) l'on récupère du 1,1,1,2,3,3-hexafluoropropane de l'autre partie de l'effluent gazeux issu du réacteur éventuellement après une étape de purification, caractérisé en ce que la température à l'entrée du réacteur est comprise entre 30 et 100°C.

Selon un mode de réalisation 2, le procédé selon le mode de réalisation 1 est caractérisé en ce que la partie de l'effluent gazeux recyclé représente au moins 90 %, de préférence entre 94 et 98 % en volume de la totalité de l'effluent à la sortie du réacteur.

Selon un mode de réalisation 3, le procédé selon le mode de réalisation 1 ou 2 est caractérisé en ce que l'effluent gazeux à la sortie du réacteur comprend de 2 à 99 % en volume du 1,1,1,2,3,3-hexafluoropropane, 0,2 à 98 % en volume d'hydrogène, de 0 à 10 % en volume de l'hexafluoropropène, de 0 à 1 % en 1,1,1,2-tetrafluoropropane et 0 à 5 % du 1,1,1,2,3-pentafluoropropane.

Selon un mode de réalisation 4, le procédé selon l'un quelconque des modes de réalisation 1 à 3 est caractérisé en ce que le catalyseur comprend du palladium, éventuellement supporté.

Selon un mode de réalisation 5, le procédé selon le mode de réalisation 4 est caractérisé en ce que le support est à base d'alumine.

Selon un mode de réalisation 6, le procédé selon l'un quelconque des modes de réalisation 1 à 5 est caractérisé en ce que le rapport molaire hydrogène/hexafluoropropène est compris entre 1 et 50, de préférence compris entre 2 et 15.

Selon un mode de réalisation 7, le procédé selon l'un quelconque des modes de réalisation 1 à 6 est caractérisé en ce que le temps de contact est compris entre 0,1 s et 20 s et de préférence compris entre 0,5 et 5 s.

Selon un mode de réalisation 8, le procédé selon l'un quelconque des modes de réalisation 1 à 7 est caractérisé en ce que la réaction d'hydrogénation est mise en oeuvre à une pression comprise entre 0,5 et 20 bar absolu et de préférence comprise entre 1 et 5 bar absolu.

Selon un mode de réalisation 9, le procédé selon l'un quelconque des modes de réalisation 1 à 8 est caractérisé en ce que la réaction d'hydrogénation est mise en oeuvre en continu.

La demanderesse a remarqué de façon surprenante que la réactivité du 1,1,1,2,3,3-hexafluoropropane dans les conditions d'hydrogénation de la présente invention est très faible. Le procédé selon la présente invention permet d'atteindre une conversion de HFP supérieure à 99 %, voire 99,5% et même supérieure à 99,8% et une sélectivité en HFC-236ea supérieure à 99%, voire 99,5 et même supérieure à 99,8%. En outre, ces performances sont stables dans le temps.

### PARTIE EXPERIMENTALE

### Exemple 1

On utilise un réacteur tubulaire en inox de diamètre interne 2,1 cm et longueur 120 cm contenant 479 g, soit 330 cm3 de catalyseur sous forme d'un lit fixe. Le catalyseur contient 0,2 % en poids de palladium supporté sur alumine α.

Pendant la durée de la réaction, on injecte en continu 1,05 mol/h d'hydrogène et 0,7 mol/h de hexafluoropropène et le débit au sein de la boucle de recyclage est de 0,480 Nm3/h (représentant un taux de recyclage de 95,3 % en volume). La pression est de 2 bar absolue. Le ratio molaire hydrogène/ HFP à l'entrée du réacteur est de 11,6 et la température en l'entrée de réacteur est de 31° C et la température maximale atteinte au cours de la réaction est de 121 ° C. Le temps de contact est de 2,28 s.

On obtient une conversion de 100% en HFP, une sélectivité de 99,39 % en HFC-236ea, de 0,53 % en HFC-245eb et de 0,07 % en HFC-254eb.

Aucune désactivation n'a été observée pendant 118h de fonctionnement.

### Exemple 2

On opère comme à l'exemple 1 sauf que la température à l'entrée du réacteur est de 81°C et la température maximale atteinte au cours de la réaction est de 157,8° C.

On obtient une conversion de 100% en HFP, une sélectivité de 98,6 % en HFC-236ea, de 1,26 % en HFC-245eb et de 0,12 % en HFC-254eb.

### Exemple 3

On utilise un réacteur tubulaire en inox de diamètre interne 2,1 cm et longueur 120 cm contenant 479 g, soit 330 cm3 de catalyseur sous forme d'un lit fixe. Le catalyseur contient 0,2 % en poids de palladium supporté sur alumine α.

Pendant la durée de la réaction, on injecte en continu 0,84 mol/h d'hydrogène et 0,7 mol/h de hexafluoropropène et le débit au sein de la boucle de recyclage est de 0,480 Nm3/h (représentant un taux de recyclage de 96,2 % en volume). La pression est de 2 bar absolue. Le ratio molaire hydrogène/ HFP à l'entrée du réacteur est de 6 et la température en l'entrée de réacteur est de 46,7° C et la température maximale atteinte au cours de la réaction est de 121,4° C. Le temps de contact est de 2,31 s.

On obtient une conversion de 100% en HFP, une sélectivité de 99,34 % en HFC-236ea, de 0,60 % en HFC-245eb et de 0,04 % en HFC-254eb.

### Exemple 4

On opère comme à l'exemple 3 sauf que l'on injecte en continu 1,4 mol/h d'hydrogène et le débit de la boucle de recyclage est de 93,9 %. Le ratio molaire hydrogène/ HFP à l'entrée du réacteur est de 17 et la température en l'entrée de réacteur est de 45,8° C et la température maximale atteinte au cours de la réaction est de 134,3° C.

On obtient une conversion de 100% en HFP, une sélectivité de 99,54 % en HFC-236ea, de 0,39 % en HFC-245eb et de 0,03 % en HFC-254eb.

## Revendications

1. Procédé de fabrication du 1,1,1,2,3,3-hexafluoropropane **caractérisé en ce que** (i) l'on fait réagir en phase gazeuse de l'hexafluoropropène avec de l'hydrogène en quantité surstoechiométrique en présence d'un catalyseur d'hydrogénation à une température comprise entre 50 et 200°C; (ii) l'on recycle au moins 90% en volume de la totalité de l'effluent gazeux issu du réacteur comprenant du 1,1,1,2,3,3-hexafluoropropane, de l'hydrogène non réagi et éventuellement de l'hexafluoropropène non réagi, du 1,1,1,2,3-pentafluoropropane et du 1,1,1,2-tetrafluoropropane et (iii) l'on récupère du 1,1,1,2,3,3-hexafluoropropane de l'autre partie de l'effluent gazeux issu du réacteur éventuellement après une étape de purification, **caractérisé en ce que** la température à l'entrée du réacteur est comprise entre 30 et 100°C.

2. Procédé selon la revendication 1 **caractérisé en ce que** la partie de l'effluent gazeux recyclé représente entre 94 et 98 % en volume de la totalité de l'effluent à la sortie du réacteur.

3. Procédé selon la revendication 1 ou 2 **caractérisé en ce que** l'effluent gazeux à la sortie du réacteur comprend de 2 à 99 % en volume du 1,1,1,2,3,3-hexafluoropropane, 0,2 à 98 % en volume d'hydrogène, de 0 à 10 % en volume de l'hexafluoropropène, de 0 à 1 % en 1,1,1,2-tetrafluoropropane et 0 à 5 % du 1,1,1,2,3-pentafluoropropane.

4. Procédé selon l'une quelconque des revendications précédentes **caractérisé en ce que** le catalyseur comprend du palladium, éventuellement supporté.

5. Procédé selon la revendication 4 **caractérisé en ce que** le support est à base d'alumine.

6. Procédé selon l'une quelconque des revendications précédentes **caractérisé en ce que** le rapport molaire hydrogène/hexafluoropropène est compris entre 1 et 50, de préférence compris entre 2 et 15.

7. Procédé selon l'une quelconque des revendications précédentes **caractérisé en ce que** le temps de contact est compris entre 0,1 s et 20 s et de préférence compris entre 0,5 et 5 s.

8. Procédé selon l'une quelconque des revendications précédentes **caractérisé en ce que** la réaction d'hydrogénation est mise en oeuvre à une pression comprise entre 0,5 et 20 bar absolu et de préférence comprise entre 1 et 5 bar absolu.

9. Procédé selon l'une quelconque des revendications précédentes **caractérisé en ce que** la réaction d'hydrogénation est mise en oeuvre en continu.

10. Procédé de fabrication du 1,1,1,2,3,3-hexafluoropropane **caractérisé en ce que** (i) l'on fait réagir en phase gazeuse de l'hexafluoropropène avec de l'hydrogène en quantité surstoechiométrique à une température comprise entre 50 et 200°C, en présence d'un catalyseur d'hydrogénation ; (ii) l'on recycle une partie de l'effluent gazeux issu du réacteur comprenant du 1,1,1,2,3,3-hexafluoropropane, de l'hydrogène non réagi et (iii) l'on récupère du 1,1,1,2,3,3-hexafluoropropane de l'autre partie de l'effluent gazeux issu du réacteur éventuellement après une étape de purification ; ledit catalyseur d'hydrogénation comprenant du palladium supporté sur de l'alumine sous la forme polymorphique α et ayant une surface spécifique supérieure à 4 m²/g.

## Patentansprüche

1. Verfahren zur Herstellung von 1,1,1,2,3,3-Hexafluorpropan, **dadurch gekennzeichnet, dass** (i) Hexafluorpropen mit Wasserstoff in einer überstöchiometrischen Menge in Gegenwart eines Hydrierkatalysators bei einer Temperatur zwischen 50 und 200 °C in der Gasphase umgesetzt wird; (ii) mindestens 90 Vol.-% der Gesamtmenge des gasförmigen Ausstroms aus dem Reaktor, der 1,1,1,2,3,3-Hexafluorpropan, nicht umgesetzten Wasserstoff und gegebenenfalls nicht umgesetztes Hexafluorpropen, 1,1,1,2,3-Pentafluorpropan und 1,1,1,2-Tetrafluorpropan enthält, zurückgeführt werden und (iii) 1,1,1,2,3,3-Hexafluorpropan des anderen Teils des gasförmigen Ausstroms aus dem Reaktor gegebenenfalls nach einem Reinigungsschritt gewonnen wird, **dadurch gekennzeichnet, dass** die Temperatur am Eingang des Reaktors zwischen 30 und 100 °C liegt.

2. Verfahren nach Anspruch 1, **dadurch gekennzeichnet, dass** der zurückgeführte Teil des gasförmigen Ausstroms zwischen 94 und 98 Vol.-% der Gesamtmenge des Ausstroms am Ausgang des Reaktors darstellt.

3. Verfahren nach Anspruch 1 oder 2, **dadurch gekennzeichnet, dass** der gasförmige Ausstrom am Ausgang des Reaktors 2 bis 99 Vol.-% 1,1,1,2,3,3-Hexafluorpropan, 0,2 bis 98 Vol.-% Wasserstoff, 0 bis 10 Vol.-% Hexafluorpropen, 0 bis 1 % 1,1,1,2-Tetrafluorpropan und 0 bis 5 % 1,1,1,2,3-Pentafluorpropan enthält.

4. Verfahren nach einem beliebigen der vorhergehenden Ansprüche, **dadurch gekennzeichnet, dass** der Katalysator Palladium umfasst, das gegebenenfalls geträgert ist.

5. Verfahren nach Anspruch 4, **dadurch gekennzeichnet, dass** der Träger auf Aluminiumoxid basiert.

6. Verfahren nach einem beliebigen der vorhergehenden Ansprüche, **dadurch gekennzeichnet, dass** das Stoffmengenverhältnis Wasserstoff/Hexafluorpropen zwischen 1 und 50, vorzugsweise zwischen 2 und 15, liegt.

7. Verfahren nach einem beliebigen der vorhergehenden Ansprüche, **dadurch gekennzeichnet, dass** die Kontaktzeit zwischen 0,1 s und 20 s und vorzugsweise zwischen 0,5 und 5 s liegt.

8. Verfahren nach einem beliebigen der vorhergehenden Ansprüche, **dadurch gekennzeichnet, dass** die Hydrierungsreaktion bei einem Druck zwischen 0,5 und 20 bar absolut und vorzugsweise zwischen 1 und 5 bar absolut durchgeführt wird.

9. Verfahren nach einem beliebigen der vorhergehenden Ansprüche, **dadurch gekennzeichnet, dass** die Hydrierungsreaktion kontinuierlich durchgeführt wird.

10. Verfahren zur Herstellung von 1,1,1,2,3,3-Hexafluorpropan, **dadurch gekennzeichnet, dass** (i) Hexafluorpropen mit Wasserstoff in einer überstöchiometrischen Menge in Gegenwart eines Hydrierkatalysators bei einer Temperatur zwischen 50 und 200 °C in der Gasphase umgesetzt wird; (ii) ein Teil des gasförmigen Ausstroms aus dem Reaktor, der 1,1,1,2,3,3-Hexafluorpropan, nicht umgesetzten Wasserstoff umfasst, zurückgeführt wird und (iii) 1,1,1,2,3,3-Hexafluorpropan des anderen Teils des gasförmigen Ausstroms aus dem Reaktor gegebenenfalls nach einem Reinigungsschritt gewonnen wird; wobei der Hydrierkatalysator Palladium umfasst, das auf Aluminiumoxid in seiner polymorphen α-Form und mit einer spezifischen Oberfläche von mehr als 4 m₂/g geträgert ist.

## Claims

1. A process for manufacturing 1,1,1,2,3,3-hexafluoropropane **characterized in that** (i) hexafluoropropene in gaseous phase is made to react with a superstoichiometric quantity of hydrogen in the presence of a hydrogenation catalyst at a temperature of between 50 and 200°C; (ii) at least 90% by volume is recycled of all of the gaseous effluent from the reactor comprising 1,1,1,2,3,3-hexafluoropropene, unreacted hydrogen and optionally unreacted hexafluoropropene, 1,1,1,2,3-pentafluoropropane and 1,1,1,2-tetrafluoropropane, and (iii) 1,1,1,2,3,3-hexafluoropropane is recovered from the other portion of the gaseous effluent from the reactor, optionally after a purification step, **characterized in that** the temperature at the input of the reactor is between 30 and 100°C.

2. The process according to claim 1, **characterised in that** the portion of the recycled gaseous effluent represents between 94 and 98% by volume of all of the effluent at the output of the reactor.

3. The process according to any one of claims 1 or 2, **characterised in that** the gaseous effluent at the output of the reactor comprises from 2 to 99% by volume of 1,1,1,2,3,3-hexafluoropropane, 0.2 to 98 % by volume of hydrogen, from 0 to 10% by volume of hexafluoropropene, from 0 to 1% of 1,1,1,2-tetrafluoropropane and 0 to 5% of 1,1,1,2,3-pentafluoropropane.

4. The process according to any one of the preceding claims, **characterised in that** the catalyst comprises palladium, optionally supported.

5. The process according to claim 4, **characterised in that** the support is alumina based.

6. The process according to any one of the preceding claims, **characterised in that** the hydrogen/hexafluoropropene molar ratio is between 1 and 50, preferably between 2 and 15.

7. The process according to any one of the preceding claims, **characterised in that** the contact time is between 0.1 s and 20 s, and preferably between 0.5 s and 5 s.

8. The process according to any one of the preceding claims, **characterised in that** the hydrogenation reaction is carried out at a pressure of between 0.5 and 20 bar absolute, and preferably between 1 and 5 bar absolute.

9. The process according to any one of the preceding claims, **characterised in that** the hydrogenation reaction is carried out continuously.

10. A process of manufacturing 1,1,1,2,3,3-hexafluoropropane, **characterised in that** (i) hexafluoropropene in gaseous phase is made to react with a superstoichiometric quantity of hydrogen at a temperature of between 50 and 200°C, in the presence of a hydrogenation catalyst; (ii) a portion is recycled of the gaseous effluent from the reactor comprising 1,1,1,2,3,3-hexafluoropropane, unreacted hydrogen, and (iii) 1,1,1,2,3,3-hexafluoropropane is recovered from the other portion of the gaseous effluent from the reactor, optionally after a purification step; the said hydrogenation catalyst comprising alumina-supported palladium in polymorphic form α and having a specific surface area greater than 4 m²/g.
